Europäisches Patentamt

European Patent Office

Office européen des brevets

(11) Publication number: **0 173 134**
**B1**

## EUROPEAN PATENT SPECIFICATION

(45) Date of publication of patent specification: 08.06.88

(51) Int. Cl.⁴: **G 01 N 1/30, C 12 Q 1/04**

(21) Application number: 85109905.1

(22) Date of filing: 06.08.85

(54) Stain for bacterial flagella.

(30) Priority: 07.08.84 JP 165232/84

(43) Date of publication of application:
05.03.86 Bulletin 86/10

(45) Publication of the grant of the patent:
08.06.88 Bulletin 88/23

(84) Designated Contracting States:
CH DE FR IT LI SE

(56) References cited:
US-A-3 554 871

VIRGINIA JOURNAL OF SCIENCE (USA), vol.
32, no. 3, page 143, US; T.L. WEAVER:
"Microbial flagella staining method or magic ?"
MANUAL OF CLINICAL MICROBIOLOGY, 3rd
edition, pages 1014-1015, American Society for
Microbiology, Washington, D.C., US.

(73) Proprietor: SHIONOGI & CO., LTD.
12, Dosho-machi 3-chome Higashi-ku
Osaka 541 (JP)

(72) Inventor: Minamide, Wakio
18-8, Matsubara-cho
Takatsuki-shi Osaka (JP)
Inventor: Nakajima, Kunihiro
739-27, Toriya-cho
Kashihara-shi Nara (JP)

(74) Representative: Vossius & Partner
Siebertstrasse 4 P.O. Box 86 07 67
D-8000 München 86 (DE)

Courier Press, Leamington Spa, England.

**Description**

Background of the invention
(1) Field of the invention
This invention relates to a stain for bacterial flagella used in identifying bacteria.

(2) Description of the prior art
There are several methods for staining bacterial flagella such as Leifson's method devised by Leifson (J. Bacteriol., *62*, 377—389 (1951)), Toda's method (A Manual of Bacteriological Exercise 5th edition edited by an alumni association of Medical Science Laboratory (Ikagakukenkyusho-gakuyukai-hen "Saikingaku-jisshu-teiyo"), Maruzen, p. 128 (1978)) and Nagao's method (Clinic and Bacteria (Rinsho To Saikin), *10*, 88—92 (1983)). The composition of each stain employed in these methods is shown below.

| Composition \ Method | Leifson's | Toda's | Nagao's | |
|---|---|---|---|---|
| Tannic acid | 1.0 | 4.0 | 1.7 | % (W/V) |
| Alum | — | 2.8 | — | % (W/V) |
| Phenol | — | 2.5 | — | % (V/V) |
| Sodium sulfate | — | — | 0.33 | % (W/V) |
| Sodium chloride | 0.5 | — | — | % (W/V) |
| Dye | 0.4 | 1.2 | 0.33 | % (W/V) |
| | Para Rosaniline acetate | Fuchsine | Night Blue | |
| | Para Rosaniline chloride | | | |
| Ethanol | ca. 33 | ca. 10 | ca. 33 | % (V/V) |
| Purified water | up to 100% | up to 100% | up to 100% | |

Clinical tests have been automatized recently, e.g. by the development and introduction of automatic machines. Tests using a simplified identification kit or an automatic or semi-automatic measuring machine have also been introduced as methods for identifying bacteria and drug sensitivity in the field of clinical bacterial tests. These methods, however, lack a test for the morphology of the bacteria.

Bacterial tests basically depend on a test for the morphology and especially tests for the morphology of the flagella, e.g. as to whether flagella are polar trichous or peritrichous, are very important for identifying bacteria. For the test for the morphology of bacteria is not often practised as a routine test, due to the following disadvantages of conventional bacterial flagella staining.

1) the preparation of the stain is not easy,
2) the stain cannot be preserved for a long time, especially at room temperature,
3) the operation of staining is difficult and it takes a long time to become skilled in it,
4) the results of staining cannot easily be judged because the results are not clear.

Summary
It is the object of this invention to provide a stain for bacterial flagella which can be stably preserved over 6 months at room temperature and does not require skilled techniques in staining and extra operation such as filtration or separation of the supernatant in preparing.

This object is attained by the stain of the present invention. The invention comprises a stain for bacterial flagella which has the following composition:

| | |
|---|---|
| a) tannic acid | 4.5—14.0 % (W/V), |
| b) alum | 1.5—4.5 % (W/V), |
| c) phenol | 1.2—3.2 % (V/V), |
| d) dye selected from Victoria Blue B, Night Blue and Gentian Violet | 0.1—0.45 % (W/V), |
| e) alkanol selected from methanol, ethanol and isopropanol | 7.0—15.0 % (V/V) |

and

| | |
|---|---|
| f) water up to | 100 % |

The stain of this invention and the staining procedure using the same, have the following advantages:

1) Preparation of this stain does not need an extra operation such as filtration (as in Toda's method) and separation of a supernatant after standing (as in Leifson's method).

2) This stain can be stably preserved at room temperature over 6 months. It is not necessary to prepare it immediately before use (as in Toda's method) and to preserve it at 4°C or lower (as in Leifson's method), because it is stable at room temperature.

3) Since this stain is stable as stated above and flagella are easily stained, skilled techniques are not necessary.

4) The time required for staining is only about 4—6 minutes when the stain is preserved at room temperature. As the staining figure is clear, this stain does not require such a complicated procedure as in Toda's method in which several slides are prepared at intervals of 30 seconds after staining and then the most preferred specimen is selected, and

5) Since the stain contains phenol, it is not necessary that the bacteria to be tested are previously fixed and sterilized with formalin (as in Nagao's and Leifson's methods).

Detailed description of the invention

The term alum in the above described composition means aluminium potassium sulfate dodecahydrate $(KAl(SO_4)_2 \cdot 12H_2O)$.

Color indexes of the dyes stated above are as follows: Victoria Blue B (44045), Night Blue (44085), and Gentian Violet (Crystal Violet) (42555). Victoria Blue is the most preferred of these dyes.

The alkanols stated above may be employed alone or as a mixture. However, it is preferable that each of them is employed alone. Good staining can be provided especially by employing isopropanol alone. Preferably purified water is employed.

A more preferred stain has the following composition:

| | |
|---|---|
| a) tannic acid | 8.0—10.0 % (W/V), |
| b) alum | 2.0—3.0 % (W/V), |
| c) phenol | 2.0—3.0 % (V/V), |
| d) dye | 0.1—0.25 % (W/V), |
| e) alkanol | 8.0—12.0 % (V/V) |

and

| | |
|---|---|
| f) water up to | 100% |

The most preferred stain has the following composition:

Stain A

| | |
|---|---|
| a) tannic acid | 9.1 % (W/V), |
| b) alum | 2.3 % (W/V), |
| c) phenol | 2.3 % (V/V), |
| d) Victoria Blue B | 0.18 % (W/V), |

and

| | |
|---|---|
| e) isopropanol | 9.0 % (V/V). |

The stain of this invention can be prepared according to usual methods. An example is shown below.

(a) A desired amount of alum is dissolved in purified water (total volume 100 ml),

(b) a desired amount of phenol is added to purified water (total volume 100 ml),

(c) a desired amount of tannic acid, and

(d) a desired amount of Victoria Blue B is dissolved in purified water (total volume 100 ml).

(c) is dissolved in a mixture of 5 ml of both (a) and (b) and then 1 ml of (d) is added thereto and the mixture is then shaken well.

While the stain of this invention can be used immediately after preparation, no change in quality of the stain is recognized after allowing it to stand for 6 months so that it can be used as it is.

The procedure of staining bacterial flagella with this stain is the same as the conventional ones except that this stain does not require treatment with formalin, as the conventional ones, for fixing and sterilizing the bacteria. An example is shown below.

3

**0 173 134**

Fresh culture incubated on a solid medium (for example MacConkey medium, Drigalski improved medium, CLED medium and Horse blood agar medium) at about 20—37°C for 16—18 hours is suspended in an appropriate amount of purified water to give a bacterial suspension. In another embodiment, fresh culture incubated in a liquid medium (especially tripticase soy broth is preferred) at about 20—37°C for 16—18 hours is used as a bacterial suspension. The bacterial suspension is dropped on a clean slide glass and dried spontaneously and then the stain is dropped thereon. When using Stain A indicated above, about 0.25 ml of it are dropped on the slide glass and the staining of flagella is completed in about 4—6 minutes if stain A is kept at room temperature. Then, the stain is washed off with water and the flagella are observed under a microscope.

With the application of cefalosporin antibiotics, it has become a serious problem that glucose-nonfermentative gram-negative rods can cause an opportunistic infection which resists the cefalosporins. The stain of this invention is very useful in identifying the gram-negative rods, since it reduces the manpower to be employed in a test for identifying bacteria, saves the kind and amount of the reagent to be used and elevates precision in the identification of the gram-negative rods.

Example

The invention is illustrated below in more detail by the following examples, which do not limit this invention.

Examples 1—3

Tannic acid as indicated in the following table is dissolved in a mixture of aqueous alum solution and aqueous phenol solution as indicated in the following table, and then the dye solution indicated in the following table is added thereto and shaken well to give a stain for bacterial flagella.

4

| Example | Ingredient | Composition | Proportion of each ingredients to total amount* |
|---|---|---|---|
| 1 | Tannic acid<br>7% aqueous alum solution<br>7% aqueous phenol solution<br>5% Night Blue ethanol solution | 0.5 g<br>5.0 ml<br>5.0 ml<br>1.0 ml | T 4.5 % (W/V)<br>M 3.2 % (W/V)<br>P 3.2 % (V/V)<br>C 0.45 % (W/V)<br>A 9.0 % (V/V) |
| 2 | Tannic acid<br>5% aqueous alum solution<br>7% aqueous phenol solution<br>2.5% Victoria Blue B methanol solution | 1.0 g<br>6.0 ml<br>4.0 ml<br>1.0 ml | T 9.1 % (W/V)<br>M 2.7 % (W/V)<br>P 2.5 % (V/V)<br>C 0.23 % (W/V)<br>A 9.1 % (V/V) |
| 3 | Tannic acid<br>5% aqueous alum solution<br>7% aqueous phenol solution<br>2.5% Gentian Violet ethanol solution | 1.5 g<br>4.5 ml<br>5.0 ml<br>1.5 ml | T 13.6 % (W/V)<br>M 2.0 % (W/V)<br>P 3.2 % (V/V)<br>C 0.34 % (W/V)<br>A 13.6 % (V/V) |
| 4 | Tannic acid<br>5% aqueous alum solution<br>5% aqueous phenol solution<br>2.0% Victoria Blue B isopropanol solution | 1.0 g<br>5.0 ml<br>5.0 ml<br>1.0 ml | T 9.1 % (W/V)<br>M 2.3 % (W/V)<br>P 2.3 % (V/V)<br>C 0.18 % (W/V)<br>A 9.0 % (V/V) |

*T=tannic acid, M=alum, P=phenol, C=dye, and A=alkanol.

0 173 134

**Claims**

1. A stain for bacterial flagella having the following composition:

| | | |
|---|---|---|
| a) tannic acid | 4.5—14.0 | % (W/V), |
| b) aluminium potassium sulfate dodecahydrate | 1.5—4.5 | % (W/V), |
| c) phenol | 1.2—3.2 | % (V/V), |
| d) dye selected from Victoria Blue B, Night Blue and Gentian Violet | 0.1—0.45 | % (W/V), |
| e) alkanol selected from methanol, ethanol and iso-propanol | 7.0—15.0 | % (V/V), |

and

| | | |
|---|---|---|
| f) water up to | 100% | |

2. The stain for bacterial flagella of claim 1, wherein said dye is Victoria Blue B.
3. The stain for bacterial flagella of claim 1, wherein said alkanol is isopropanol.

**Patentansprüche**

1. Färbemittel für Bakteriengeißeln und mit folgender Zusammensetzung:

| | | |
|---|---|---|
| a) Gerbsäure | 4,5—14,0 | % (Gew./Vol.) |
| b) Kalium-Aluminiumsulfat-Dodekahydrat | 1,5—4,5 | % (Gew./Vol.) |
| c) Phenol | 1,2—3,2 | % (Vol./Vol.) |
| d) Farbstoff aus der Gruppe Viktoria-blau B, Nacutblau und Gentiana-violett | 0,1—0,45 | %(Gew./Vol.) |
| e) Alkanol aus der Gruppe Methanol, Äthanol und Isopropanol | 7,0—15,0 | % (Vol./Vol.) |

und

| | | |
|---|---|---|
| f) Wasser auf | 100% | |

2. Färbemittel nach Anspruch 1, wobei der Farbstoff Viktoriablau B ist.
3. Färbemittel nach Anspruch 1, wobei das Alkanol Isopropanol ist.

**Revendications**

1. Un colorant pour les flagelles bactériens ayant la composition suivante:

| | | |
|---|---|---|
| a) acide tannique | 4,5—14,0 | % (poids/vol.), |
| b) le dodécahydrate desulfate de potassium et d'aluminium | 1,5—4,5 | % (poids/vol.), · |
| c) phénol | 1,2—3,2 | % (vol./vol.), |
| d) colorant choisi parmi le bleu de Victoria B, le bleu nuit et le Gentiane violet | 0,1—0,45 | % (poids/vol.), |
| e) alcanol choisi parmi le méthanol, l'éthanol et l'isopropanol | 7,5—15,0 | % (vol./vol.), |

et

| | | |
|---|---|---|
| f) eau jusqu'à | 100% | |

2. Le colorant pour les flagelles bactériens de la revendication 1 où ledit colorant est le bleu de Victoria B.

3. Le colorant pour les flagelles bactériens de la revendication 1 où ledit alcanol est l'isopropanol.